# EUROPEAN PATENT APPLICATION

(11) **EP 3 769 773 A1**
(43) Date of publication of application: **27.01.2021**
(21) Application number: 20187518.4
(22) Date of filing: 23.07.2020
(51) Int. Cl.: A61K 36/064, A61K 8/00, A61K 9/00, A61P 1/02, A61P 31/02

(54) **COMPOSITIONS AND METHODS FOR TREATING THE ORAL CAVITY**

(30) Priority: 23.07.2019 US 201962877395 P
(71) Applicant: JOHNSON & JOHNSON CONSUMER INC., Skillman, NJ 08558 (US)
(72) Inventor: LI, Wen-Hwa Ting, Skillman, NJ New Jersey 08558 (US); MAHMOOD,, Khalid, Skillman, NJ New Jersey 08558 (US); PARSA, Ramine, Skillman, NJ New Jersey 08558 (US)
(74) Representative: Kirsch, Susan Edith

(57) **Abstract**

The present invention relates to compositions comprising one or more compounds and/or extracts which induce, promote and/or improve production/release/delivery/excretion of mucin from and/or in the oral cavity, and methods of using the compositions to treat the oral cavity.

## Description

### FIELD OF THE INVENTION

The present invention relates to compositions comprising one or more compounds and/or extracts which induce, promote and/or improve production/release/delivery/excretion of mucin from and/or in the oral cavity, and methods of using the compositions to treat the oral cavity.

### BACKGROUND OF THE INVENTION

Mucus coatings form a protective barrier for the mucosal tissues of the oral cavity. Mucins, a gel forming component of mucus, play an important role play an important role mucosal barrier protection.

As noted by Frenkel and Ribbeck, "[e]ach of the salivary mucins MUC5B, MUC7, MUC19, MUC1, and MUC4 are composed of a unique domain structure that influences the mucins' physical properties and localization in the oral cavity." Frenkel ES, Ribbeck K., Salivary mucins in host defense and disease prevention, J Oral Microbiol. 2015; 7: 29759.

Frenkel and Ribbeck further note that, "MUC5B is the primary gel-forming mucin in the mouth that is secreted by mucous cells in the submandibular, sublingual, palatine, and labial salivary glands. Transcripts and glycoproteins of MUC19, another gel-forming salivary mucin, have been identified, but MUC5B is still thought to be the predominate gel-forming mucin in the oral cavity. MUC7 is also a secreted mucin that exists primarily as monomers or dimers and lacks gel-forming properties. These monomers and dimers are able to self-associate, however, to form higher order assemblies that could be important for bacterial aggregation. MUC7 localization within salivary glands varies between individuals; it has been identified in mucous cells of submandibular and sublingual glands, but the presence of MUC7 in serous cells of these glands is variable. MUC1 and MUC4 are membrane-associated mucins that line the ducts of parotid, submandibular, and minor salivary glands. These mucins may play a role in cell signal transduction, improved hydration and could form scaffolds for secreted mucins to bind, leading to improved prevention of decalcification of teeth (See JP-A-62-212317), oral mucosal protection and reduced periodontitis and dental caries.

More specifically, MUC1 has been implicated in playing a role in numerous oral conditions (Kashyap B, Kullaa AM. Regulation of mucin 1 expression and its relationship with oral diseases. Archives of Oral Biology. 2020; 117: 104791). It has been suggested that subjects with decreased salvation (xerostomia or dry mouth) and reduced levels of MUC1 expression in their oral epithelial cells may increase chances of oral diseases (Chang, W. I., Chang, J. Y., Kim, Y. Y., Leeb, G., & Kho, H. S. MUC1 expression in the oral mucosal epithelial cells of the elderly. Archives of Oral Biology. 2011; 56, 885-890). MUC1's may also have a role in hydrating the oral surface (Sengupta A, Valdramidou D, Huntley S et al: Distribution of MUC1 in the normal human oral cavity is localized to the ducts of minor salivary glands. Arch Oral Biol, 2001; 46: 529-38 and Pramanik R, Osailan SM, Challacombe SJ et al: Protein and mucin retention on oral mucosal surfaces in dry mouth patients. Eur J Oral Sci, 2010; 118: 245-53).

Furthermore, MUC1 has been suggested to play a role in the regulation of inflammation which may lead to oral conditions such as gingivitis and periodontitis. Importantly, toll-like receptors TLRs are known to play important role in the detection of pathogens and the initiation of immune responses (Akira, S., & Takeda, K. Toll-like receptor signaling. Nature Reviews Immunology, 2004; 4(7), 449-511.). Recently, Ueno K et. Al. were able to show that MUC1 can negatively regulate TLR signaling (Ueno, K., Koga, T., Kato, K., Golenbock, D. T., Gendler, S. J., Kai, H., & Kim, K. C. MUC1 mucin is a negative regulator of toll-like receptor signaling. American Journal of Respiratory Cell and Molecular Biology. 2008; 38, 263-268.) which may help to dampen overactive inflammatory responses that drive tissue destruction in gingivitis and gum disease or periodontal diseases.

Similarly, decreased levels of MUC4 have been associated with periodontitis (Annual review of selected scientific literature: A report of the Committee on Scientific Investigation of the American Academy of Restorative Dentistry. Journal of Prosthetic Dentistry 2008 (Dec.) pages 816-877. and Lundmark A, Johannsen G, Eriksson K, Kats A, Jansson L, Tervahartiala T, et al. Mucin 4 and matrix metalloproteinase 7 as novel salivary biomarkers for periodontitis. J Clin Periodontol 2017;44:247-54.)

There is therefore a need for composition that would promote and/or improve the production and/or release of mucin from and/or in the oral cavity.

The present inventors have discovered extracts, or sources of extracts, of the genus *Pichia* which can induce, promote and/or improve production/release/delivery/excretion of mucin from and/or in the oral cavity.

Accordingly, an aspect of the present invention relates to oral care compositions comprising one or more extracts, or sources of extracts, of the genus *Pichia.*

A further aspect of the present invention relates to oral care compositions comprising a safe and effective amount of one or more extracts, or sources of extracts, of the genus *Pichia* to induce, promote and/or improve production/release/delivery/excretion of mucin from and/or in the oral cavity.

### SUMMARY OF THE INVENTION

Accordingly, the present invention relates to a composition comprising:
i) one or more extracts, or sources of extracts, of the genus *Pichia;* and
ii) a pharmaceutically (or cosmetically) acceptable carrier.

The composition of the invention is suitable for treating the oral cavity.

The present invention also relates to a composition for use in treating oral disease wherein the composition comprises:
i) one or more extracts, or sources of extracts, of the genus *Pichia;* and
ii) a pharmaceutically (or cosmetically) acceptable carrier.

The present invention also relates to methods for producing/releasing/delivering/excreting mucin from and/or in the oral cavity comprising the step of administering (optionally, in a patient in need of such production/release/deliverance/excretion of mucin) a composition of the invention.

The composition may optionally comprise a safe and effective amount of a permeation enhancer.

The present invention also relates to a non-therapeutic method of treating the oral cavity or a cosmetic method using a composition comprising:
i) one or more extracts, or sources of extracts, of the genus *Pichia;* and
ii) a pharmaceutically or cosmetically acceptable carrier.

### DETAILED DESCRIPTION OF INVENTION

It is believed that one skilled in the art can, based upon the description herein, utilize this invention to its fullest extent. The following specific embodiments can be construed as merely illustrative, and not limitative of the remainder of the disclosure in any way whatsoever.

The compositions of the present invention can comprise, consist of, or consist essentially of the elements, steps and limitations of the invention described herein, as well any of the additional or optional ingredients, components, or limitations described herein.

The term "comprising" (and its grammatical variations) as used herein is used in the inclusive sense of "having" or "including" and not in the exclusive sense of "consisting only of."

The terms "a" and "the" as used herein are understood to encompass the plural as well as the singular.

Unless defined otherwise, all technical and scientific terms used herein have the same meaning as commonly understood by one of ordinary skill in the art to which the invention belongs. Also, all publications, patent applications, patents, and other references mentioned herein are incorporated by reference in their entirety to the extent that they are not inconsistent with this specification. As used herein, all percentages are by weight of the total composition unless otherwise specified

As used herein, a composition that is "essentially free" of an ingredient means the composition that has about 2% or less of that ingredient by weight based on the total weight of the composition. Preferably, a composition that is essentially free of an ingredient has about 1% or less, more preferably about 0.5% or less, more preferably about 0.1% or less, more preferably about 0.05 or less, more preferably about 0.01% or less by weight based on the total weight of composition of the ingredient. In certain more preferred embodiments, a composition that is essentially free of an ingredient is free of the ingredient, i.e. has none of that ingredient in the composition.

As used herein, the term "safe and effective amount" means an amount of disclosed the extract, compound or of the composition sufficient to induce, promote and/or improve the production/release/delivery/excretion of mucin from and/or in the oral cavity, but low enough to avoid serious side effects. The safe and effective amount of the compound, extract, or composition will vary with e.g. the age, health and environmental exposure of the end user, the duration and nature of the treatment, the specific extract, ingredient, or composition employed, the particular pharmaceutically (or cosmetically) acceptable carrier utilized, and like factors.

In certain embodiments, the present invention as disclosed herein may be practiced in the absence of any compound or element (or group of compounds or elements) which is not specifically disclosed herein.

In general, IUPAC nomenclature rules are used herein and according to the following term definitions.

The term "C₁₋₈ alkyl," whether used alone or as part of a substituent group, refers to a saturated aliphatic branched or straight-chain monovalent hydrocarbon radical having from 1-8 carbon atoms. For example, "C₁₋₈alkyl" specifically includes the radicals methyl, ethyl, 1-propyl, 2-propyl, 1-butyl, 2-butyl, tert-butyl, 1-butyl, 1-pentyl, 2-pentyl, 3-pentyl, 1-hexyl, 2-hexyl, 3- hexyl, 1-heptyl, 2-heptyl, 3-heptyl, 1-octyl, 2-octyl, 3-octyl and the like. Said term may also refer to the corresponding alkyldiyl radical. Alkyl and alkyldiyl radicals may be attached to a core molecule via a terminal carbon atom or via a carbon atom within the chain. Similarly, any number of substituent variables may be attached to an alkyl or alkyldiyl radical when allowed by available valences.

The term "C₁₋₄alkyl," whether used alone or as part of a substituent group, refers to a saturated aliphatic branched or straight-chain monovalent hydrocarbon radical or alkyldiyl linking group having a specified number of carbon atoms, wherein the radical is derived by the removal of one hydrogen atom from a carbon atom and the alkyldiyl linking group is derived by the removal of one hydrogen atom from each of two carbon atoms in the chain. The term "C₁₋₄alkyl" refers to a radical having from 1-4 carbon atoms in a linear or branched arrangement. For example, "C₁₋₄alkyl" specifically includes the radical's methyl, ethyl, 1-propyl, 2-propyl, 1-butyl, 2-butyl, tert- butyl, 1-butyl, and the like. Alkyl and alkyldiyl radicals may be attached to a core molecule via a terminal carbon atom or via a carbon atom within the chain. Similarly, any number of substituent variables may be attached to an alkyl or alkyldiyl radical when allowed by available valences.

The term "C₂₋₄alkenyl" refers to an alkenyl radical having from 2-4 carbon atoms. For example, specifically includes the radicals ethenyl, propenyl, allyl (2-propenyl), butenyl and the like. As described above, an alkenyl radical may be similarly attached to a core molecule and further substituted where indicated.

The term "halo" as such or in combination with other terms means halogen atom, such as fluoro, chloro, bromo or iodo.

The term "substituted," refers to a core molecule in which one or more hydrogen atoms have been replaced with that amount of substituents allowed by available valences. Substitution is not limited to the core molecule, but may also occur on a substituent radical, whereby the radical becomes a linking group.

The term "independently selected" refers to two or more substituents that may be selected from a substituent variable group, wherein the selected substituents may be the same or different.

The term "dependently selected" refers to one or more substituent variables that are specified in an indicated combination for substitution in a core molecule (e.g. variables that refer to groups of substituents appearing in a tabular list of compounds).

Acceptable salts from inorganic bases include, for example, sodium or potassium salts, and the like. Acceptable salts from organic bases include, for example, salts formed with primary, secondary, or tertiary amines, and the like.

### Compounds And/Or Extracts which Induce, Promote and/or Improve

### Production/Release/Delivery/Excretion of Mucin in the Oral Cavity.

The present invention comprises one or more compounds and/or extracts which induce, promote and/or improve production/release/delivery/excretion of mucin from and/or in the oral cavity.

In certain embodiments, the compounds and/or extracts which induce, promote and/or improve production/release/delivery/excretion of mucin from and/or in the oral cavity are, or comprise, extracts, or sources of extracts, of the genus *Pichia.*

*Pichia* is a genus of yeasts in the family *Saccharomycetaceae.* More than 100 species of this genus are known. Suitable species for use in the compositions of the present invention include (selected from or selected from the group consisting of) *Pichia anomala, Pichia guilliermondii, Pichia norvegensis,* and *Pichia ohmeri. Pichia anomala* (formerly named *Hansenula anomala*) can be found in raw milk and cheese. The extracts of yeasts of the genus *Pichia* are rich in mannans, polysaccharides composed of mannose monomers. Extracts or sources of extracts of the genus *Pichia* may be isolated from the fruit or other aerial parts of a plant. Any pharmaceutically or cosmetically acceptable extract of the genus of *Pichia* may be used. Mixtures of extracts, or sources of extract, of the above species from the genus of *Pichia* may also be used.

In certain embodiments, the extracts, or sources of extracts, from genus of *Pichia* used in the present invention are extracts of *Pichia anomala.* Extracts of *Pichia anomala* can be isolated from the fruit or other aerial parts of a plant. In certain embodiments, a suitable extract of *Pichia anomala* is produced from a strain of *Pichia anomala* present on fruit or leaves of a kiwi plant. In another embodiment, extract of *Pichia anomala* is commercially available from Silab-France (St. Viance, France) as PRO-LIPISKIN, or UNFLAMAGYL®, where the extract is produced from a strain of *Pichia anomala* present on sugar cane. In certain embodiments, the *Pichia anomala* extract is sold under the tradename Hyalurodine also supplied by Silab-France.

In one embodiment, the *Pichia anomala* extract is obtained in accordance with the following process as described in FR2897266 and FR2938768, both of which are incorporated by herein by reference.

In certain embodiments, the extraction process described below eliminates much of the protein from a *Pichia* extract and concentrates the active in terms of mannan. The process comprises at least one enzymatic hydrolysis step of the proteins, to obtain peptides and small proteins and, in certain embodiments, a further step of removing these small peptides and proteins by filtration based on the selection of the size of such molecules.

In certain embodiments, the extracts of *Pichia* genus, including *Pichia anomala* extract, are obtained by an extraction process involving one or more hydrolysis enzyme (s) to hydrolyze proteins in *Pichia* genus, either successively or simultaneously.

In certain embodiments, enzymatic hydrolysis is used to break-down proteins in the extract of the *Pichia* genus into protein fractions having weight average molecular weights of less than 5000Da. Suitable hydrolysis enzymes include, but is not limited to, at least one peptidase, in certain embodiments, chosen from papain, trypsin, chymotrypsin, subtilisin, pepsin, thermolysin, pronase, flavastacine, enterokinase, factor Xa protease, Turin, bromelain, proteinase K, genenase I, thermitase, carboxypeptidase A, carboxypeptidase B, collagenase or mixtures thereof.

In certain embodiments, the enzymes used to obtain the *Pichia* genus extract are inactivated prior to separation of the resulting soluble and insoluble phases.

In one embodiment, the *Pichia anomala* extract is characterized as having:
- a solids content of between 5 and 300 g/l,
- a pH between 4 and 9,
- a protein content between 2 and 170 g/l, and
- a sugars content ranging between 1 and 100 g/l

In certain embodiments, extract of *Pichia* genus comprises a mannan content of greater than or equal to 30% of the total weight of dried *Pichia* genus extract, or optionally at a mannan content of at least 50% by weight of the total weight of the dried *Pichia* genus extract.

In certain embodiments, the phase of *Pichia* genus extract is dried (and the solids content is measured) by passing the *Pichia* genus extract containing phase through oven heat of 105 °C (or about 105 °C) in the presence of sand until a constant weight is obtained/observed.

In certain embodiments, the solids content of the dried extract of *Pichia* genus is between 10 and 200 g/l, or optionally between 26 and 40 g/l.

In certain embodiments, the pH measured by the potentiometric method at room temperature (25 °C) leads to values of between 4.5 and 8.5, optionally between 6.0 and 7.0.

Determination of total sugar content, the DUBOIS method can be used. In the presence of concentrated sulphuric acid and phenol, reducing sugars give an orange yellow compound. From a standard range, the total sugar content of a sample can be determined. In certain embodiments, the total sugar content in the dried extract of *Pichia* genus is between 7 and 145 g/l, or optionally between 18 and 29 g/l. In certain embodiments, the dried extract of *Pichia* genus contains at least 30% of total sugars by weight of dried extract of *Pichia* genus compared to the total solids by weight of dried extract of *Pichia* genus, optionally, at least 50% by weight of dried extract of *Pichia* genus.

In certain embodiments, the carbohydrate fraction of the dried extract of *Pichia* genus is composed of mannose and glucose in the form of (or essentially in the form of) oligosaccharides and polysaccharides having a weight average molecular weight of from about 180 to about 800,000 Da, optionally, from about 5000 to about 515,000 Da, optionally from about 6000 to about 270,000 Da. In certain embodiments, at least 70% (or about 70%), optionally at least 75% (or about 75%), at least 80% (or about 80%), optionally at least 85% (or about 85%), at least 90% (or about 90%), optionally at least 95% (or about 95%), or 100% (or about 100%) of the oligosaccharides and polysaccharides in the dried extract of *Pichia* genus fall within the aforementioned weight average molecular weight ranges.

Determination of the protein content protein is obtained by the Kjedhal method. In certain embodiments, the dried extract of *Pichia* genus has a protein content of between 4 and 90 g/l, or optionally between 12 and 18 g/l. The dried extract of *Pichia* genus contains less than 45% of proteins, or optionally less than 30%, of the total solids in the dried extract of *Pichia* genus.

In certain embodiments, the dried extract of *Pichia* genus comprises mannans, mannoses polymerized in the form of oligosaccharides and polysaccharides, whose weight average molecular weight is from about 180 to about 800,000 Da, optionally, from about 5000 to about 515,000 Da, optionally from about 6000 to about 270,000 Da. dried extract of *Pichia* genus. In certain embodiments, at least 70% (or about 70%), optionally at least 75% (or about 75%), at least 80% (or about 80%), optionally at least 85% (or about 85%), at least 90% (or about 90%), optionally at least 95% (or about 95%), or 100% (or about 100%) of the oligosaccharides and polysaccharides in the dried extract of *Pichia* genus fall within the aforementioned weight average molecular weight ranges.

In certain embodiments, the extraction process includes a step, after the step of enzymatic hydrolysis of proteins, of removing (e.g., through filtration) proteins having a weight average molecular weight of less than 5000Da. Accordingly, the extracts of the *Pichia* genus are free of or substantially free of proteins and/or peptides having a weight average molecular weight of less than 5000Da.

In certain embodiments, the above described extraction process may include a step of deodorizing, bleaching and / or stabilizing the extract of *Pichia* genus before the filtrations. The filtrations can be the following: - Press filtration, and - sterilizing filtration.

In certain embodiments, the extracts used belong to the variety *Pichia anomala.* A particular non-limiting example of a production process is described below.
- Extracts (or yeasts) of *Pichia anomala* are cultured in a culture medium adapted to their development, then centrifuged to recover the biomass,
- The biomass is then milled in a ball mill. Then the ground material is resuspended in water at a concentration of 50 grams per liter before enzymatic hydrolysis in basic medium at 30 °C for 6 hours,
- After hydrolysis, the product is centrifuged and filtered before sterilization,
- By successive filtrations on filters of different sizes, a hydrolyzate containing at least 30% of mannans relative to the total weight of the solids is obtained and/or proteins of specific weight average molecular weight are obtained. (The hydrolyzate obtained is in the form of a clear liquid aqueous solution of light yellow color.)

In certain embodiments, the *Pichia anomala* extract is obtained in accordance with the following manufacturing examples:
1. *Pichia anomala* extract A:
   I. Extraction of the *Pichia anomala* extract A:
      Preparation of *Pichia anomala* extract A includes the following steps:
      - culture of yeast *Pichia anomala* in an environment adapted to their development,
      - centrifugation to retrieve the biomass,
      - solubilization of biomass,
      - enzymatic hydrolysis at basic pH,
      - separation of the soluble and insoluble phases,
      - heat treatment,
      - filtration, and
      - sterile filtration.
   II. Characterization of the *Pichia anomala* extract A:
      The *Pichia anomala* extract A obtain above is characterized by:
      - - a solids content of between 48 and 84 g/l,
      - - a pH between 4 and 9,
      - - a protein content between 19 and 48 g/l, and
      - - a total sugar content between 10 and 42 g/l.
2. *Pichia anomala* extract B
   I. Extraction of the *Pichia anomala* extract B:
      Preparation of *Pichia anomala* extract B includes the following steps:
      - culture of yeast *Pichia anomala* in an environment adapted to their development,
      - centrifugation to retrieve the biomass,
      - solubilization of biomass,
      - enzymatic hydrolysis at acid pH,
      - separation of the soluble and insoluble phases,
      - heat treatment,
      - filtration, and
      - sterile filtration.
   II. Characterization of the *Pichia anomala* extract B:
      The *Pichia anomala* extract B obtain above is characterized by:
      - - a solids content of between 58 and 95 g/l,
      - - a pH between 4 and 9,
      - - a protein content between 23 and 54 g/l, and
      - - a total sugar content between 12 and 32 g/l.
3. *Pichia anomala* extract C
   I. Extraction of the *Pichia anomala* extract C:
      Preparation of *Pichia anomala* extract C includes the following steps:
      - culture of yeast *Pichia anomala* in an environment adapted to their development,
      - centrifugation to retrieve the biomass,
      - solubilization of biomass,
      - successive enzymatic hydrolyses in basic medium,
      - separation of the soluble and insoluble phases,
      - heat treatment,
      - filtration, and
      - sterile filtration.
   II. Characterization of the *Pichia anomala* extract C:
      The *Pichia anomala* extract C obtain above is characterized by:
      - - a solids content of between 91 and 195 g/l,
      - - a pH between 4 and 9,
      - - a protein content between 36 and 111 g/l, and
      - - a total sugar content between 18 and 65 g/l.
4. *Pichia anomala* extract D
   I. Extraction of the *Pichia anomala* extract D:
      Preparation of *Pichia anomala* extract D includes the following steps:
      - culture of yeast *Pichia anomala* in an environment adapted to their development,
      - centrifugation to retrieve the biomass,
      - solubilization of biomass,
      - hydrolyzed simultaneously with at least two enzymes at acid pH,
      - separation of the soluble and insoluble phases,
      - heat treatment,
      - filtration, and
      - sterile filtration.
   II. Characterization of the *Pichia anomala* extract D:
      The *Pichia anomala* extract D obtain above is characterized by:
      - - a solids content of between 5 and 53 g/l,
      - - a pH between 4 and 9,
      - - a protein content between 2 and 30 g/l, and
      - - a total sugar content between 1 and 18 g/l.
5. *Pichia anomala* extract E
   I. Extraction of the *Pichia anomala* extract E:
      Preparation of *Pichia anomala* extract E includes the following steps:
      - culture of yeast *Pichia anomala* in an environment adapted to their development,
      - centrifugation to retrieve the biomass,
      - solubilization of biomass in a hydroglycolique environment,
      - hydrolyzed simultaneously with at least two enzymes at acid pH,
      - separation of the soluble and insoluble phases,
      - heat treatment,
      - filtration, and
      - sterile filtration.
   II. Characterization of the *Pichia anomala* extract E:
      The *Pichia anomala* extract E obtain above is characterized by:
      - - a solids content of between 172 and 300 g/l,
      - - a pH between 4 and 9,
      - - a protein content between 69 and 170 g/l, and
      - - a total sugar content between 34 and 100 g/l.

In certain embodiments, the extract, or source of extracts, of the genus *Pichia* comprises oligosaccharides and polysaccharides having an average degree of polymerization of from DP 1 to DP 4444, optionally from DP 30 to DP 2860, optionally from DP 35 to DP 1500. In certain embodiments, at least 70% (or about 70%), optionally at least 75% (or about 75%), at least 80% (or about 80%), optionally at least 85% (or about 85%), at least 90% (or about 90%), optionally at least 95% (or about 95%), or 100% (or about 100%) of the oligosaccharides and polysaccharides in the dried extract of *Pichia* genus fall within the aforementioned average degree of polymerization ranges.

In certain embodiments, the extract, or source of extracts, of the genus *Pichia* are present in the compositions of the present invention so as to provide a *Pichia* extract concentration in, or contacting, oral mucosa or tissue cells in the user, after topical application, of at least 0.3mg/ml (or about 0.3 mg/ml), optionally, at least 0.5 mg/ml (or about 0.5 mg/ml), optionally, at least 1 mg/ml (or about 1 mg/ml), optionally, at least 1.5 mg/ml (or about 1.5mg/ml), optionally, at least 2 mg/ml (or about 2 mg/ml), optionally, at least 2.5 mg/ml (or about 2.5mg/ml), optionally, at least 3mg/ml (or about 3mg/ml), optionally, at least 3.5 mg/ml (or about 3.5mg/ml), optionally, at least 4 mg/ml (or about 4 mg/ml), optionally, at least 4.5 mg/ml (or about 4.5 mg/ml), optionally, at least 5 mg/ml (or about 5 mg/ml), or at least 5.5 mg/ml (or about 5.5 mg/ml), optionally, at least 6 mg/ml (or about 6 mg/ml), or optionally, at least 6.5 mg/ml (or about 6.5 mg/ml), optionally, at least 7 mg/ml (or about 7 mg/ml), optionally, at least 7.5 mg/ml (or about 7.5 mg/ml), optionally, at least 8 mg/ml (or about 8 mg/ml), optionally, at least 8.5 mg/ml (or about 8.5 mg/ml), optionally, at least 9 mg/ml (or about 9 mg/ml), optionally, at least 8.5 mg/ml (or about 8.5 mg/ml), optionally, at least 9 mg/ml (or about 9 mg/ml), optionally, at least 9.5 mg/ml (or about 9.5 mg/ml), or optionally, at least 10 mg/ml (or about 10mg/ml) to 100 mg/ml (or about 100 mg/ml), optionally, to 95 mg/ml (or about 95 mg/ml), optionally, to 90 mg/ml (or about 90 mg/ml), optionally, to 85 mg/ml (or about 85mg/ml), optionally, to 80 mg/ml (or about 80 mg/ml), optionally, to 75 mg/ml (or about 75 mg/ml), optionally, to 70 mg/ml (or about 70 mg/ml), optionally, to 65 mg/ml (or about 65 mg/ml), optionally, to 60 mg/ml (or about 60 mg/ml), optionally, to 55 mg/ml (or about 55 mg/ml), optionally, to 50 mg/ml (or about 50 mg/ml), optionally, to 45 mg/ml (or about 45 mg/ml), optionally, to 40 mg/ml (or about 40 mg/ml), optionally, to 35 mg/ml (or about 35 mg/ml), optionally, to 30 mg/ml (or about 30 mg/ml), optionally, to 25 mg/ml (or about 25 mg/ml), optionally, to 20 mg/ml (or about 20 mg/ml), or optionally, to 15 mg/ml (or about 15 mg/ml).

In certain embodiments, the extract, or source of extracts, of the genus *Pichia* are present in the compositions of the present invention at a concentration of from 0.01% (or about 0.01%), optionally, from 0.05% (or about 0.05%), optionally, from 0.1% (or about 0.1%), optionally, from 0.5% (or about 0.5%), optionally, from 1% (or about 1%), optionally, from 1.5% (or about 1.5%), optionally, from 2% (or about 2%), optionally, from 2.5% (or about 2.5%), optionally, from 3% (or about 3%), optionally, from 3.5%, (or about 3.5%), optionally, from 4% (or about 4%), optionally, from 4.5% (or about 4.5%), optionally, from 5% (or about 5%), optionally, from 5.5% (or about 5.5%), optionally, from 6% (or about 6%), optionally, from 6.5% (or about 6.5%), optionally, from 7% (or about 7%), optionally, from 7.5% (or about 7.5%), optionally, from 8% (or about 8%), optionally, from 8.5% (or about 8.5%), optionally, from 9% (or about 9%), optionally, from 9.5% (or about 9.5%), optionally, from 10% (or about 10%), optionally, from 10.5% (or about 10.5%), optionally, from 11% (or about 11%), optionally, from 11.5% (or about 11.5%), optionally, from 12% (or about 12%), optionally, from 12.5% (or about 12.5%), optionally, from 13% (or about 13%), optionally, from 13.5% (or about 13.5%), optionally, from 14% (or about 14%), optionally, from 14.5% (or about 14.5%), optionally, from 15% (or about 15%), optionally, from 15.5% (or about 15.5%), optionally, from 16% (or about 16%), optionally, from 16.5% (or about 16.5%), optionally, from 17% (or about 17%), optionally, from 17.5% (or about 17.5%), optionally, from 18% (or about 18%), optionally, from 18.5% (or about 18.5%), optionally, from 19% (or about 19%), optionally, from 19.5% (or about 19.5%), optionally, from 20% (or about 20%), optionally, from 20.5% (or about 20.5%) to 30% (or about 30%), optionally, to 35% (or about 35%), optionally, to 40% (or about 40%), optionally, to 45% (or about 45%), optionally, to 50% (or about 50%), optionally, to 55% (or about 55%), optionally, to 60% (or about 60%), optionally, to 65% (or about 65%), optionally, to 70% (or about 70%), optionally, to 75% (or about 75%), optionally, to 80% (or about 80%), optionally, to 85% (or about 85%), optionally, to 90% (or about 90%), optionally, to 95% (or about 95%), or optionally, to 100% (or about 100%), by weight, of the total composition.

### Permeation Enhancer

In certain embodiments, the compositions of the present invention optionally comprise a permeation enhancer. Permeation enhancers are those substances which promote the absorption of drug through the skin, mucosal or cellular membranes temporarily by transiently enhancing the skin, mucosal or cellular membrane permeability.

Suitable permeation enhancers include (selected from or selected from the group consisting of) either alone or in combination, surfactants such as saponins, polyoxyethylene, polyoxyethylene ethers of fatty acids such as polyoxyethylene 4-, 9-, 10-, and 23-lauryl ether, polyoxyethylene 10-and 20-cetyl ether, polyoxyethylene 10-and 20-stearyl ether, sorbitan monooleate, sorbitan monolaurate, polyoxyethylene monolaurate, polyoxyethylene sorbitans such as polyoxyethylene sorbitan monolaurate, decamethonium, decamethonium bromide, and dodecyltrimethylammonium bromide; chelators such natural polyacids (e.g., citric acid), phosphate salts (e.g., disodium pyrophosphate), phosphonates, bisphosphonates (e.g., etridronic acid), aminocarboxylic acids (e.g., ethylenediaminetetraacetic acid (EDTA) and disodium EDTA) and ethylenediamine-N,N'-disuccinic acid (EDDS)); bile salts and acids such as cholic acid, deoxycholic acid, glycocholic acid, glycodeoxycholic acid, taurocholic acid, taurodeoxycholic acid, sodium cholate, sodium glycocholate, glycocholate, sodium deoxycholate, sodium taurocholate, sodium glycodeoxycholate, sodium taurodeoxycholate, chenodeoxycholic acid, and urosdeoxycholic acid; fusidic acid derivatives, glycyrrhizic acid, and ammonium glycyrrhizide, with saponin EDTA, fusidic acid, polyoxyethylene 9-lauryl ether, polyoxyethylene 20-stearylether, glycocholate, or mixtures of any of the above.

The concentration of permeation enhancer administered should be the minimum amount needed to sufficiently increase absorption of the compound and/or extract through the mucous or other barrier membranes of the oral cavity. Generally, concentrations ranging from 0.01% (or about 0.01%), optionally, from 0.05% (or about 0.05%), optionally, from 0.1% (or about 0.1%), optionally, from 0.15% (or about 0.15%), optionally, from 0.2% (or about 0.2%), optionally, from 0.25% (or about 0.25%) to 2% (or about 2%), optionally, to 2.5% (or about 2.5%), optionally, to 3% (or about 3%), optionally, to 3.5%, (or about 3.5%), optionally, to 4% (or about 4%), optionally, to 4.5% (or about 4.5%), optionally, to 5% (or about 5%), optionally, to 5.5% (or about 5.5%), optionally, to 6% (or about 6%), optionally, to 6.5% (or about 6.5%), optionally, to 7% (or about 7%), optionally, to 7.5% (or about 7.5%), optionally, to 8% (or about 8%), optionally, to 8.5% (or about 8.5%), optionally, to 9% (or about 9%), optionally, to 9.5% (or about 9.5%), optionally, to 10% (or about 10%), optionally, to 10.5% (or about 10.5%), optionally, to 11% (or about 11%), optionally, to 11.5% (or about 11.5%), optionally, to 12% (or about 12%), optionally, to 12.5% (or about 12.5%), optionally, to 13% (or about 13%), optionally, to 13.5% (or about 13.5%), optionally, to 14% (or about 14%), optionally, to 14.5% (or about 14.5%), optionally, to 15% (or about 15%), optionally, to 15.5% (or about 15.5%), optionally, to 16% (or about 16%), optionally, to 16.5% (or about 16.5%), optionally, to 17% (or about 17%), optionally, to 17.5% (or about 17.5%), optionally, to 18% (or about 18%), optionally, to 18.5% (or about 18.5%), optionally, to 19% (or about 19%), optionally, to 19.5% (or about 19.5%), optionally, to 20% (or about 20%), of the total composition (w/v), are useful in the compositions of the present invention.

### Pharmaceutically or Cosmetically Acceptable Carrier

In certain preferred embodiments, the compositions of the present invention are compositions for oral care, including, for example, oral care compositions in the form of a solution, mouthwash, mouth rinse, mouth spray, toothpaste, tooth gel, sub-gingival gel, mousse, foam, denture care product, dentifrice, lozenge, chewable tablet, dissolvable tablet, dry powder and the like. The oral care composition may also be incorporated into or onto floss, dissolvable strips or films or integrated into or onto a device or applicator for oral use.

In certain embodiments, the compositions of the invention comprise the one or more compounds and/or extracts which induce, promote and/or improve production/release/delivery/excretion of mucin from and/or in the oral cavity and a vehicle. Any suitable vehicle may be used in the compositions of the present invention. Preferably, the vehicle is selected from the group consisting of cosmetically-acceptable and pharmaceutically-acceptable vehicles. As used herein, "cosmetically-acceptable" and "pharmaceutically-acceptable" vehicles are liquid, solid, or other ingredients suitable for use as vehicles in products for mammals, including humans without undue toxicity, incompatibility, instability, irritation, allergic response, and the like.

For liquid compositions, the vehicle may be any suitable aqueous or non-aqueous liquid vehicle. In certain embodiments, the liquid vehicle comprises water. For example, in many compositions, as will be understood by those of skill in the art, water is added to q.s. (Quantum Sufficit, Latin for "as much as needed") the composition. In certain embodiments, the composition comprises from about 60% to about 99.99% water, including from about 70% to about 95% water, from about 80% to 95% water, from about 60% to about 90% water, from about 60% to about 80% water, or from about 60% to about 75% water.

In certain embodiments, alcohol may, optionally, be added to the composition. Any of a variety of alcohols represented by the formula R₄-OH, wherein R₄ is an alkyl group having from 2 to 6 carbons, may be used in the present invention. Examples of suitable alcohols of formula R₄-OH include ethanol; n-propanol, iso-propanol; butanols; pentanols; hexanols, and combinations of two or more thereof, and the like. In certain embodiments, the alcohol is, or comprises, ethanol.

In some embodiments, the alcohol may be present in the composition in an amount of at least about 10.0% v/v of the total composition, or from about 10% to about 35% v/v of the total composition, or from about 15% to about 30% v/v of the total composition and may be from about 20% to about 25% v/v of the total composition.

In some embodiments, the composition may comprise a reduced level of alcohol. The phrase "reduced level" of alcohol means an amount of a R₄-OH alcohol of about 10% v/v or less, optionally of about 5% v/v or less, optionally of about 1% v/v or less, optionally of about 0.1% v/v or less by volume of the total composition. In certain embodiments, the compositions of the present invention are free of R₄-OH alcohols.

Alternatively, the compositions of the present invention may be formulated in a dissolvable tablet, dry powder, chewing gum, film, semi-solid, solid or liquid concentrate form. In such embodiments, for example, water is added to q.s. as necessary in the case of liquid dissolvable tablet, concentrates or powdered formulations, or water may be removed using standard evaporation procedures known in the art to produce a composition in dry powder form. Evaporated, or freeze-dried forms are advantageous for storage and shipping.

The compositions of the present invention may further comprise any of a variety of optional ingredients therein, including, but not limited to oily components, active ingredients, additional surfactants, humectants, solvents, flavors, sweeteners, colorants, preservatives, pH adjusters, pH buffers, and the like.

Any of a variety of oily components may be used in the present compositions. The oily component may comprise any one or more oils, or other materials that are water insoluble, or substantially water-insoluble, meaning that its solubility is less than about 1% by weight in water at 25°C or, optionally, less than about 0.1%. In certain embodiments, the oily component of the present invention comprises, consists essentially of, or consists of, at least one essential oil, i.e. a natural or synthetic (or combination thereof) concentrated hydrophobic material of vegetable origin, generally containing volatile compounds, at least one flavor oil, or a combination of two or more thereof. Examples of suitable essential oils, flavor oils, and their amounts are described below. In certain embodiments, the composition comprises a total amount of oily component of about 0.05% w/w or more, about 0.1% w/w or more, or about 0.2% w/w or more of oily component.

In certain embodiments, compositions of the present invention comprise essential oils. Essential oils are volatile aromatic oils which may be synthetic or may be derived from plants by distillation, expression or extraction, and which usually carry the odor or flavor of the plant from which they are obtained. Useful essential oils may provide antiseptic activity. Some of these essential oils also act as flavoring agents. Useful essential oils include but are not limited to citra, thymol, menthol, methyl salicylate (wintergreen oil), eucalyptol, carvacrol, camphor, anethole, carvone, eugenol, isoeugenol, limonene, osimen, n-decyl alcohol, citronel, a-salpineol, methyl acetate, citronellyl acetate, methyl eugenol, cineol, linalool, ethyl linalool, saffola vanillin, spearmint oil, peppermint oil, lemon oil, orange oil, sage oil, rosemary oil, cinnamon oil, pimento oil, laurel oil, cedar leaf oil, gerianol, verbenone, anise oil, bay oil, benzaldehyde, bergamot oil, bitter almond, chlorothymol, cinnamic aldehyde, citronella oil, clove oil, coal tar, eucalyptus oil, guaiacol, tropolone derivatives such as hinokitiol, lavender oil, mustard oil, phenol, phenyl salicylate, pine oil, pine needle oil, sassafras oil, spike lavender oil, storax, thyme oil, tolu balsam, turpentine oil, clove oil, and combinations thereof.

In certain embodiments, the essential oils are selected from the group consisting of thymol ((CH₃)₂CHC₆H₃(CH₃)OH, also known as isopropyl-m-cresol), eucalyptol (C₁₀H₁₈O, also known as cineol), menthol (CH₃C₆H₉(C₃H₇)OH), also known as hexahydrothymol), methyl salicylate (C₆H₄OHCOOCH₃, also known as wintergreen oil), isomers of each of these compounds, and combinations of two or more thereof. In some embodiments, the compositions of the present invention contain thymol. In some embodiments, the compositions of the present invention contain menthol. In some embodiments, the composition contains all four of these essential oils.

In certain embodiments, thymol is employed in amounts of from about 0.0001% to about 0.6% w/v, or from about 0.005% to about 0.07% w/v of the composition. In certain embodiments, eucalyptol may be employed in amounts of from about 0.0001% to about 0.51 w/v, or from about 0.0085% to about 0.10% w/v of the composition. In certain embodiments, menthol is employed in amounts of from about 0.0001% to about 0.25% w/v, or from about 0.0035% to about 0.05% w/v of the composition. In certain embodiments, methyl salicylate is employed in amounts of from about 0.0001% to about 0.28% w/v, or from about 0.004% to about 0.07% w/v of the composition. In certain embodiments, the total amount of all of such essential oils present in the disclosed compositions can be from about 0.0004% to about 1.64% w/v, or from about 0.0165% to about 0.49% w/v of the composition.

In certain embodiments, fluoride providing compounds may be present in the mouth rinse compositions of this invention. These compounds may be slightly water soluble or may be fully water soluble and are characterized by their ability to release fluoride ions or fluoride containing ions in water. Typical fluoride providing compounds are inorganic fluoride salts such as soluble alkali metal, alkaline earth metal, and heavy metal salts, for example, sodium fluoride, potassium fluoride, ammonium fluoride, cupric fluoride, zinc fluoride, stannic fluoride, stannous fluoride, barium fluoride, sodium hexafluorosilicate, ammonium hexafluorosilicate, sodium fluorozirconate, sodium monofluorophosphate, aluminum mono-and difluorophosphate and fluorinated sodium calcium pyrophosphate. Amine fluorides, such as N'-octadecyltrimethylendiamine-N,N,N'- tris(2-ethanol)-dihydrofluoride and 9-octadecenylamine-hydrofluoride), may also be used. In certain embodiments, the fluoride providing compound is generally present in an amount sufficient to release up to about 5%, or from about 0.001% to about 2%, or from about 0.005% to about 1.5% fluoride by weight of the composition.

In certain embodiments, sensitivity reducing agents, such as potassium salts of nitrate and oxalate in an amount from about 0.1% to about 5.0% w/v of the composition may be incorporated into the present invention. Other potassium releasing compounds are feasible (e.g. KCl). High concentrations of calcium phosphates may also provide some added sensitivity relief. These agents are believed to work by either forming an occlusive surface mineral deposit on the tooth surface or through providing potassium to the nerves within the teeth to depolarize the nerves. A more detailed discussion of suitable sensitivity reducing agents can be found in US 2006/0013778 to Hodosh and U.S. Pat. No. 6,416,745 to Markowitz et al., both of which are herein incorporated by reference in their entirety.

In certain embodiments, compounds with anti-calculus benefits (e.g. various carboxylates, polyaspartic acid, etc.) may be incorporated into the present invention. Also useful as an anticalculus agent are the anionic polymeric polycarboxylates. Such materials are well known in the art, being employed in the form of their free acids or partially or preferably fully neutralized water soluble alkali metal (e.g. potassium and preferably sodium) or ammonium salts. Preferred are 1:4 to 4:1 by weight copolymers of maleic anhydride or acid with another polymerizable ethylenically unsaturated monomer, preferably methyl vinyl ether (methoxyethylene) having a molecular weight (M.W.) of about 30,000 to about 1,000,000. These copolymers are available, for example, as Gantrez 25 AN 139 (M.W. 500,000), AN 119 (M.W. 250,000) and preferably S-97 Pharmaceutical Grade (M.W. 70,000), of GAF Chemicals Corporation.

Additional anti-calculus agents may be selected from the group consisting of polyphosphates (including pyrophosphates) and salts thereof polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; polyvinyl phosphonates and salts thereof; polyolefin phosphonates and salts thereof; polypeptides; and mixtures thereof; carboxy-substituted polymers; and mixtures thereof. In one embodiment, the salts are alkali metal or ammonium salts. Polyphosphates are generally employed as their wholly or partially neutralized water-soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation, which are commercially known as Sodaphos (n≈6), Hexaphos (n≈13), and Glass H (n≈21, sodium hexametaphosphate), and mixtures thereof. The pyrophosphate salts useful in the present invention include, alkali metal pyrophosphates, di-, tri-, and mono-potassium or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate (Na₂H₂P₂O₇), dipotassium pyrophosphate, tetrasodium pyrophosphate (Na₄P₂O₇), tetrapotassium pyrophosphate (K₄P₂O₇), and mixtures thereof. Polyolefin sulfonates include those wherein the olefin group contains 2 or more carbon atoms, and salts thereof. Polyolefin phosphonates include those wherein the olefin group contains 2 or more carbon atoms. Polyvinylphosphonates include polyvinylphosphonic acid. Diphosphonates and salts thereof include azocycloalkane-2,2-diphosphonic acids and salts thereof, ions of azocycloalkane-2,2-diphosphonic acids and salts thereof, azacyclohexane-2,2-diphosphonic acid, azacyclopentane-2,2-diphosphonic acid, N-methyl-azacyclopentane-2,3-diphosphonic acid, EHDP (ethane-1-hydroxy-1,1,-diphosphonic acid), AHP (azacycloheptane-2,2-diphosphonic acid), ethane-1-amino-1,1-diphosphonate, dichloromethane-diphosphonate, etc. phosphonoalkane carboxylic acid or their alkali metal salts include PPTA (phosphonopropane tricarboxylic acid), PBTA (phosphonobutane-1,2,4-tricarboxylic acid), each as acid or alkali metal salts. Polyolefin phosphates include those wherein the olefin group contains 2 or more carbon atoms. Polypeptides include polyaspartic and polyglutamic acids.

In certain embodiments, zinc salts such as zinc chloride, zinc acetate or zinc citrate may be added as an astringent for an "antiseptic cleaning" feeling, as a breath protection enhancer or as anti-calculus agent in an amount of from about 0.0025% w/v to about 0.75% w/v of the composition.

Any of a variety of additional surfactants may be used in the present invention. Suitable surfactants may include anionic, non-ionic, cationic, amphoteric, zwitterionic surfactants, and combinations of two or more thereof. Examples of suitable surfactants are disclosed, for example, in U.S. Pat. No. 7,417,020 to Fevola, et al which is incorporated in its entirety herein by reference.

In certain embodiments, the compositions of the present invention comprise a non-ionic surfactant. Those of skill in the art will recognize that any of a variety of one or more non-ionic surfactants include, but are not limited to, compounds produced by the condensation of alkylene oxide groups (hydrophilic in nature) with an organic hydrophobic compound which may be aliphatic or alkyl-aromatic in nature. Examples of suitable nonionic surfactants include, but are not limited to, alkyl polyglucosides; alkyl glucose amines, block copolymers such as ethylene oxide and propylene oxide copolymers e.g. poloxamers; ethoxylated hydrogenated castor oils available commercially for example under the trade name CRODURET (Croda Inc., Edison, NJ); alkyl polyethylene oxide e.g. polysorbates, and/or; fatty alcohol ethoxylates; polyethylene oxide condensates of alkyl phenols; products derived from the condensation of ethylene oxide with the reaction product of propylene oxide and ethylene diamine; ethylene oxide condensates of aliphatic alcohols; long chain tertiary amine oxides; long chain tertiary phosphine oxides; long chain dialkyl sulfoxides; and mixtures thereof.

Exemplary non-ionic surfactants are selected from the group known as poly(oxyethylene)-poly(oxypropylene) block copolymers. Such copolymers are known commercially as poloxamers and are produced in a wide range of structures and molecular weights with varying contents of ethylene oxide. These non-ionic poloxamers are non-toxic and acceptable as direct food additives. They are stable and readily dispersible in aqueous systems and are compatible with a wide variety of formulations and other ingredients for oral preparations. These surfactants should have an HLB (Hydrophilic-Lipophilic Balance) of between about 10 and about 30 and preferably between about 10 and about 25. By way of example, non-ionic surfactants useful in this invention include the poloxamers identified as poloxamers 105, 108, 124, 184, 185, 188, 215, 217, 234, 235, 237, 238, 284, 288, 333, 334, 335, 338, 407, and combinations of two or more thereof. In certain preferred embodiments, the composition comprises poloxamer 407.

In certain embodiments, the compositions of the claimed invention comprise less than about 9% of non-ionic surfactant, less than 5%, or less than 1.5%, or less than 1%, or less than 0.8, less than 0.5%, less than 0.4%, or less than .3% of non-ionic surfactants. In certain embodiments, the composition of the present invention is free of non-ionic surfactants.

In certain embodiments, the compositions of the present invention also contain at least one alkyl sulfate surfactant. In certain embodiments, suitable alkyl sulfate surfactants include, but are not limited to sulfated C₈ to C₁₈, optionally sulfated C₁₀ to C₁₆ even numbered carbon chain length alcohols neutralized with a suitable basic salt such as sodium carbonate or sodium hydroxide and mixtures thereof such that the alkyl sulfate surfactant has an even numbered C₈ to C₁₈, optionally C₁₀ to C₁₆, chain length. In certain embodiments, the alkyl sulfate is selected from the group consisting of sodium lauryl sulfate, hexadecyl sulfate and mixtures thereof. In certain embodiments, commercially available mixtures of alkyl sulfates are used. A typical percentage breakdown of alkyl sulfates by alkyl chain length in commercially available sodium lauryl sulfate (SLS) is as follows:

| | |
|---|---|
| Alkyl | Component |
| Chain | Percentage |
| Length | in SLS |

| | |
|---|---|
| C₁₂ | >60% |
| C₁₄ | 20%-35% |
| C₁₆ | <10% |
| C₁₀ | <1% |
| C₁₈ | <1% |

In certain embodiments, the alkyl sulfate surfactant is present in the composition from about 0.001% to about 6.0% w/v, or optionally from about 0.1% to about 0.5% w/v of the composition.

Another suitable surfactant is one selected from the group consisting of sarcosinate surfactants, isethionate surfactants and taurate surfactants. Preferred for use herein are alkali metal or ammonium salts of these surfactants, such as the sodium and potassium salts of the following: lauroyl sarcosinate, myristoyl sarcosinate, palmitoyl sarcosinate, stearoyl sarcosinate and oleoyl sarcosinate. The sarcosinate surfactant may be present in the compositions of the present invention from about 0.1% to about 2.5%, or from about 0.5% to about 2% by weight of the total composition.

Zwitterionic synthetic surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate.

The amphoteric surfactants useful in the present invention include, but are not limited to, derivatives of aliphatic secondary and tertiary amines in which the aliphatic radical can be a straight chain or branched and wherein one of the aliphatic substituents contains from about 8 to about 18 carbon atoms and one contains an anionic water-solubilizing group, e.g., carboxylate, sulfonate, sulfate, phosphate, or phosphonate. Examples of suitable amphoteric surfactants include, but are not limited alkylimino-diproprionates, alky lamphoglycinates (mono or di), alkylamphoproprionates (mono or di), alkylamphoacetates (mono or di), N-alkyl [3-aminoproprionic acids, alkylpolyamino carboxylates, phosphorylated imidazolines, alkyl betaines, alkylamido betaines, alkylamidopropyl betaines, alkyl sultaines, alkylamido sultaines, and mixtures thereof. In certain embodiments, the amphoteric surfactant is selected from the group consisting of alkylamidopropyl betaines, amphoacetates such as sodium auroamphoacetate and mixtures thereof. Mixtures of any of the above-mentioned surfactants can also be employed. A more detailed discussion of anionic, nonionic and amphoteric surfactants can be found in Us. Pat. No. 7,087,650 to Lennon; U.S. Pat. No. 7,084,104 to Martin et al.; U.S. Pat. No. 5,190,747 to Sekiguchi et al.; U.S. Pat. No. 4,051,234 to Gieske, et al. and U.S. Pat. No. 10,434,050 to Gambogi et al., each of which patents are herein incorporated by reference in their entirety.

In certain embodiments, the compositions of the claimed invention comprise less than about 9% of amphoteric surfactant, less than 5%, or less than 1.5%, or less than 1%, or less than 0.8, less than 0.5%, less than 0.4%, or less than .3% of amphoteric surfactants. In certain embodiments, the composition of the present invention is free of amphoteric surfactants.

Additional surfactants may be added with the alkyl sulfate surfactant to aid in solubilization of the essential oils provided such surfactants do not affect the bioavailability of the essential oils. Suitable examples include additional anionic surfactants, nonionic surfactants, amphoteric surfactants and mixtures thereof. However, in certain embodiments, the total surfactant concentration (including the alkyl sulfate surfactant alone or in combination with other surfactants) for mouth rinses of the present invention should not exceed or should about 9% or less, optionally, the total surfactant concentration should be about 5% or less, optionally about 1% or less, optionally about 0.5% or less w/w% of active surfactant by weight of the composition.

In certain embodiments, a sugar alcohol (humectant) is also added to the oral compositions of the present invention. The sugar alcohol solvent(s) may be selected from those multi-hydroxy-functional compounds that are conventionally used in oral and ingestible products. In certain embodiments, the sugar alcohol (s) should be nonmetabolized and non-fermentable sugar alcohol (s). In specific embodiments, the sugar alcohols include, but are not limited to sorbitol, glycerol, xylitol, mannitol, maltitol, inositol, allitol, altritol, dulcitol, galactitol, glucitol, hexitol, iditol, pentitol, ribitol, erythritol and mixtures thereof. Optionally, the sugar alcohol is selected from the group consisting of sorbitol and xylitol or mixtures thereof. In some embodiments, the sugar alcohol is sorbitol. In certain embodiments, the total amount of sugar alcohol (s), which are added to effectively aid in the dispersion or dissolution of the mouth rinse or other ingredients, should not exceed about 50% w/ of the total composition. Or, total amount of sugar alcohol should not exceed about 30% w/v of the total composition. Or, total amount of sugar alcohol should not exceed 25% w/v of the total composition. The sugar alcohol can be in an amount of from about 1.0% to about 24% w/v, or from about 1.5% to about 22% w/v, or from about 2.5% to about 20% w/v of the total composition.

In certain embodiments, a polyol solvent is added to the composition. The polyol solvent comprises a polyol or polyhydric alcohol selected from the group consisting of polyhydric alkanes (such as propylene glycol, glycerin, butylene glycol, hexylene glycol, 1,3-propanediol); polyhydric alkane esters (dipropylene glycol, ethoxydiglycol); polyalkene glycols (such as polyethylene glycol, polypropylene glycol) and mixtures thereof. In certain embodiments, the polyol solvent can be present in an amount of from 0% to about 40% w/v, or from about 0.5% to about 20% w/v, or from about 1.0% to about 10% w/v of the composition.

In certain embodiments, the compositions of the present invention have a pH of about 11 or less. In some embodiments, the compositions have a pH of from about 3 to about 7, or from about 3.5 to about 6.5, or from about 3.5 to about 5.0.

As will be recognized by those of skill in the art, the pH of the composition may be adjusted or maintained using a buffer in an amount effective to provide the composition with a pH at or below 11. The composition can optionally comprise at least one pH modifying agents among those useful herein include acidifying agents to lower pH, basifying agents to raise pH, and buffering agents to maintain pH within a desired range. For example, one or more compounds selected from acidifying, basifying and buffering agents can be included to provide a pH of about 2 to about 7, or in various embodiments from about 3 to about 6, or from about 4 to about 5. Any orally acceptable pH modifying agent can be used including without limitation hydrochloric, carboxylic and sulfonic acids, acid salts (e.g., monosodium citrate, disodium citrate, monosodium malate, etc.), alkali metal hydroxides such as sodium hydroxide, borates, silicates, imidazole and mixtures thereof. One or more pH modifying agents are optionally present in a total amount effective to maintain the composition in an orally acceptable pH range. In certain embodiments, inorganic acids may be used as the buffer added to the composition.

In certain embodiments, organic acids may be used as the buffer added to the composition. Organic acids suitable for use in the compositions of the present invention include, but are not limited to, ascorbic acid, sorbic acid, citric acid, glycolic acid, lactic acid and acetic acid, benzoic acid, salicylic acid, phthalic acid, phenolsulphonic acid, and mixtures thereof, optionally, the organic acid is selected from the group consisting of benzoic acid, sorbic acid, citric acid and mixtures thereof, or optionally, the organic acid is benzoic acid.

Generally, the amount of buffering compound is from about 0.001% to about 20.0% of the composition. In certain embodiment, the organic acid buffer is present in amounts of from about 0.001% to about 10% w/v of the composition, or from about 0.01% to about 1% of the composition.

In certain embodiments, additional conventional components may be added as in mouthwashes and mouth rinses of the prior art. Whereas some alcohol containing mouth rinses have a pH of about 7.0, reduction of the alcohol level may require the addition of acidic preservatives, such as sorbic acid or benzoic acid, which reduce pH levels. Buffer systems are then necessary to control the pH of the composition at optimal levels. This is generally accomplished through the addition of a weak acid and its salt or a weak base and its salt. In some embodiments, useful systems have been found to be sodium benzoate and benzoic acid in amounts of from 0.01% (or about 0.01% w/v) to 1.0% w/v (or about 1.0% w/v) of the composition, and sodium citrate and citric acid in amounts of from 0.001% (or about 0.001% w/v) to 1.0% w/v (or about 1.0% w/v) of the composition, phosphoric acid and sodium/potassium phosphate of amounts from 0.01% (or about 0.01%) to 1.0% (or about 1.0%) by weight of the composition. In certain embodiments, the buffers are incorporated in amounts that maintain the pH at levels of from 3.0 (or about 3.0) to 8.0 (or about 8.0), optionally from 3.5 (or about 3.5) to 6.5 (or about 6.5), optionally from 3.5 (or about 3.5) to 5.0 (or about 5.0).

Additional buffering agents include alkali metal hydroxides, ammonium hydroxide, organic ammonium compounds, carbonates, sesquicarbonates, borates, silicates, phosphates, imidazole, and mixtures thereof. Specific buffering agents include monosodium phosphate, trisodium phosphate, sodium hydroxide, potassium hydroxide, alkali metal carbonate salts, sodium carbonate, imidazole, pyrophosphate salts, sodium gluconate, sodium lactate, citric acid, and sodium citrate.

Sweeteners such as aspartame, sodium saccharin (saccharin), sucralose, stevia, acesulfame K and the like may be added for better taste in amounts of from about 0.0001% w/v to about 1.0% w/v. In certain preferred embodiments, the sweetener comprises sucralose.

In certain embodiments, the composition further comprises flavors or flavorants to modify or magnify the taste of the composition or reduce or mask the sharp "bite" or "burn" of ingredients such as thymol. Suitable flavors include, but are not limited to, flavor oils such as oil of anise, anethole, benzyl alcohol, spearmint oil, citrus oils, vanillin and the like may be incorporated. Other flavors such as citrus oils, vanillin and the like may be incorporated to provide further taste variations. In these embodiments, the amount of flavor oil added to the composition can be from about 0.001% to about 5% w/v, or from about 0.01% to about 0.3% w/v of the total composition. The particular flavors or flavorants, and other taste improving ingredients, employed will vary depending upon the particular taste and feel desired. Those skilled in the art can select and customize these types of ingredients to provide the desired results.

In certain embodiments, acceptably approved food dyes may be used to provide a pleasing color to the compositions of the invention. These may be selected from, but not limited to, the long list of acceptable food dyes. Suitable dyes for this purpose include FD&C yellow #5, FD&C yellow #10, FD&C blue #1 and FD&C green #3. These are added in conventional amounts, typically in individual amounts of from about 0.00001% w/v to about 0.0008% w/v, or from about 0.000035% w/v to about 0.0005% w/v of the composition.

Other conventional ingredients may be used in the liquid or mouth rinse compositions of this invention, including those known and used in the art. Examples of such ingredients include thickeners, suspending agents and softeners. Thickeners and suspending agents useful in the compositions of the present invention can be found in US Pat. 5,328,682 to Pullen et al.*,* herein incorporated by reference in its entirety. In certain embodiments, these are incorporated in amounts of from about 0.1% w/v to about 0.6% w/v, or about 0.5% w/v of the composition.

In some embodiments, antimicrobial preservatives may be added to the composition. Some antimicrobial preservatives which may be used include , but are not limited to cationic antibacterials, such as sodium benzoate, polyquaternium polycationic polymers (i.e. polyquaternium-42: Poly[oxyethylene(dimethylimino)ethylene(dimethylimino)ethylene dichloride]), quaternary ammonium salts or quaternary ammonium compounds, parabens (i.e. parahydroxybenzoates or esters of parahydroxybenzoic acid), hydroxyacetophenone, 1,2-Hexanediol, Caprylyl Glycol, chlorhexidine, alexidine, hexetidine, benzalkonium chloride, domiphen bromide, cetylpyridinium chloride (CPC), tetradecylpyridinium chloride (TPC), N-tetradecyl-4-ethylpyridinium chloride (TDEPC), octenidine, bisbiguanides, zinc or stannous ion agents, grapefruit extract, and mixtures thereof. Other antibacterial and antimicrobial agents include, but are not limited to: 5-chloro-2-(2,4-dichlorophenoxy)-phenol, commonly referred to as triclosan; 8-hydroxyquinoline and its salts, copper II compounds, including, but not limited to, copper(II) chloride, copper(II) sulfate, copper(II) acetate, copper(II) fluoride and copper(II) hydroxide; phthalic acid and its salts including, but not limited to those disclosed in U.S. Pat. No. 4,994,262, including magnesium monopotassium phthalate; sanguinarine; salicylanilide; iodine; sulfonamides; phenolics; delmopinol, octapinol, and other piperidino derivatives; niacin preparations; nystatin; apple extract; thyme oil; thymol; antibiotics such as augmentin, amoxicillin, tetracycline, doxycycline, minocycline, metronidazole, neomycin, kanamycin, cetylpyridinium chloride, and clindamycin; analogs and salts of the above; methyl salicylate; hydrogen peroxide; metal salts of chlorite; pyrrolidone ethyl cocoyl arginate; lauroyl ethyl arginate monochlorohydrate; and mixtures of all of the above. In another embodiment, the composition comprises phenolic antimicrobial compounds and mixtures thereof Antimicrobial components may be present from about 0.001% to about 20% by weight of the oral care composition. In another embodiment the antimicrobial agents generally comprise from about 0.1% to about 5% by weight of the oral care compositions of the present invention.

Other antibacterial agents may be basic amino acids and salts. Other embodiments may comprise arginine.

Other useful oral care actives and/or inactive ingredients and further examples thereof can be found in US patents 6,682,722 to Majeti et al. and 6,121,315 to Nair et al., each of which are herein incorporated by reference in its entirety.

In certain methods of the present invention comprise treating a condition or disease of the oral cavity, including the teeth, mucosal membranes/gums, and the like, by applying to the oral cavity, or injecting into the oral cavity or otherwise into the mammalian body, a compound or composition of the claimed invention. In certain methods of the present invention the oral disease is selected from decalcification of teeth, periodontitis and dental caries.

The compounds and compositions of the present invention may be used in a variety of methods of treating a mammalian body, in particular for disrupting a biofilm on a surface of the oral cavity. According to certain embodiments, the present invention comprises disrupting biofilm on a surface by contacting the surface comprising biofilm with a composition of the present invention. In certain embodiments, the present invention comprises removing biofilm from a surface by contacting the surface comprising biofilm with a composition of the present invention. In certain embodiments, the present invention comprises reducing bacterial attachment to a surface by contacting the surface with a composition of the present invention. In certain embodiments, the present method comprises inhibiting plaque by contacting a surface of the oral cavity with a compound or composition of the present invention.

Any suitable surface of the oral cavity may be contacting in accord with the methods of the present invention including one or more surfaces selected from the group consisting of surfaces of one or more teeth, surfaces of the gums, combinations of two or more thereof, and the like.

In each of the above methods, the composition of the claimed method may be introduced to the surface to be contacted via any of a variety of methods. In certain embodiments, the composition is introduced into the oral cavity and applied to the surface by a user as a mouthwash or mouth rinse. In certain embodiments, the composition is introduced to the oral cavity and applied to the surface as a toothpaste on an article for cleaning the teeth, e.g. a toothbrush. The compositions of the present invention may be further introduced via the mouth and applied to the surface as a gum, lozenge, dissolvable strip, or the like.

Furthermore, the contacting step of the methods of the present invention may comprise contacting the surface with the composition for any suitable amount of time. In certain embodiments, the contacting step comprises contacting the surface for less than thirty seconds. In certain embodiments, the contacting step comprises contacting the surface with the composition for thirty seconds or more, for example, for about thirty seconds, for about 40 seconds, for about one minute, or for greater than one minute.

The composition and products containing such compositions of this invention may be prepared using methodology that is well known by an artisan of ordinary skill.

### Examples

Any compositions of the present invention as described in following examples illustrate specific embodiments of compositions of the present invention, but are not intended to be limiting thereof. Other modifications can be undertaken by the skilled artisan without departing from the spirit and scope of this invention.

Oral care formulations can be prepared containing one or more compounds and/or extract which induce, promote and/or improve production/release/delivery/excretion of mucin from and/or in the cornea as shown in Example 1.

### Example 1

Tables 1 and 2 illustrate the components of such formulations (as illustrated in formulations 1 and 15), which components can be incorporated as described below using conventional mixing technology.

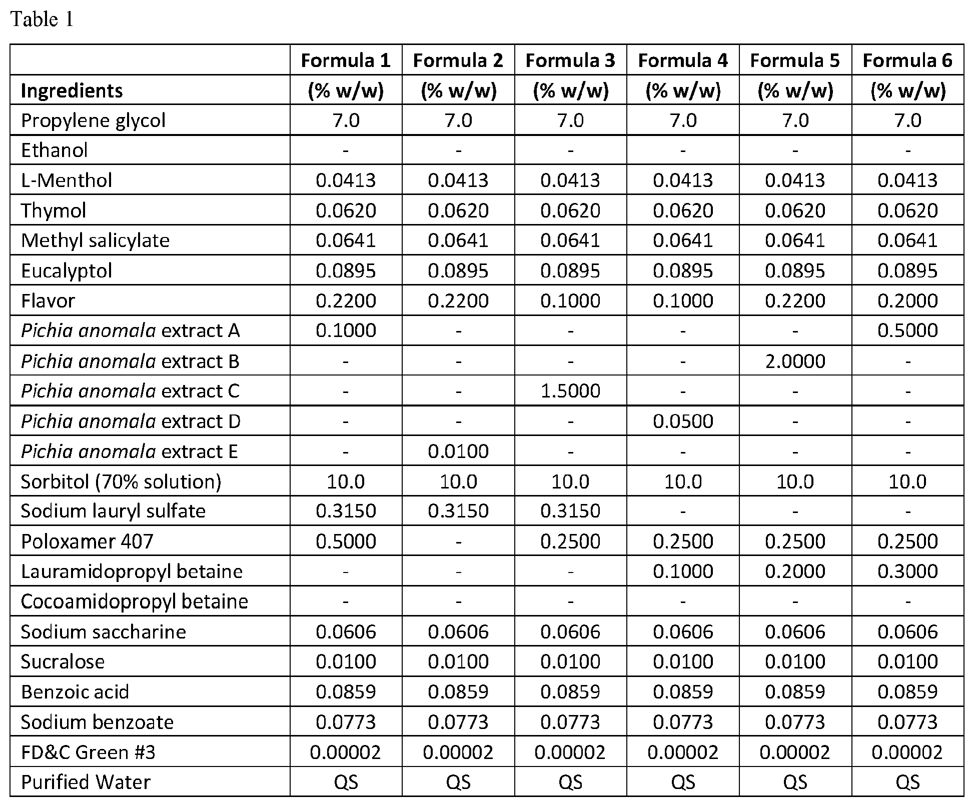

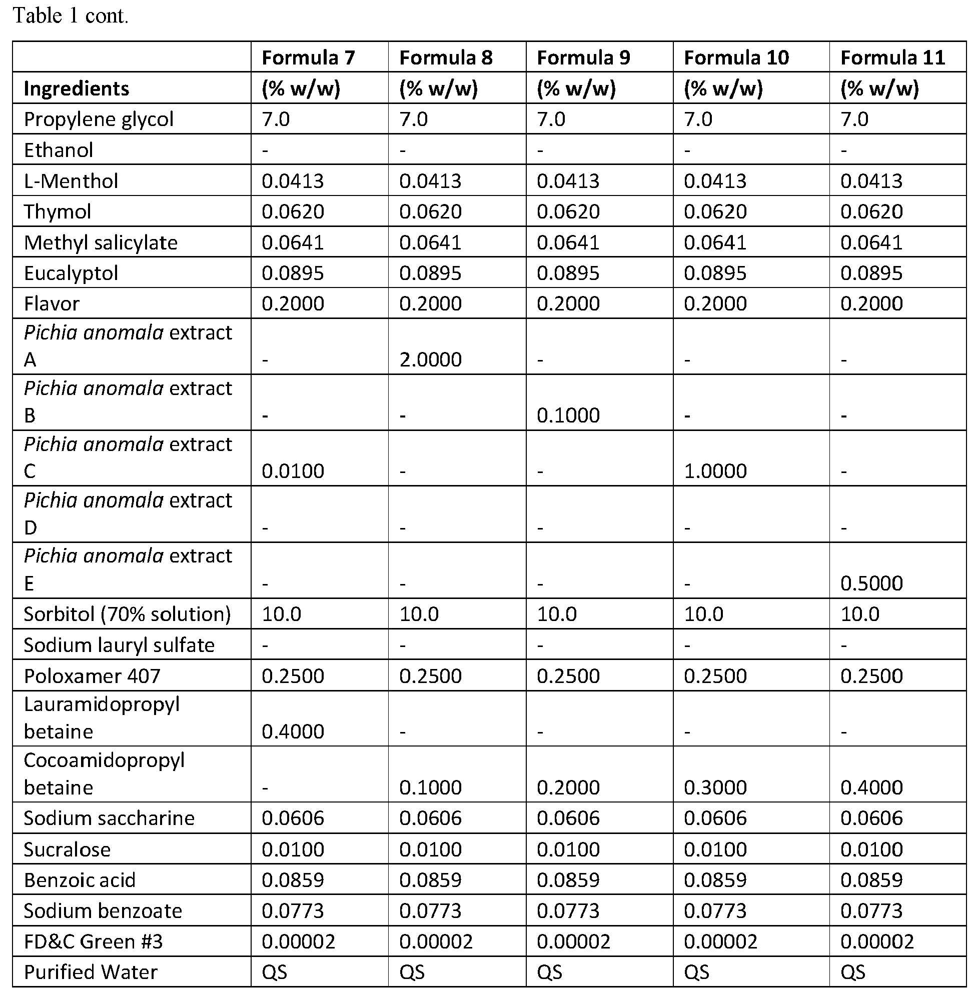

### Methods for making formulations of Table 1

Formulations 1-11 can be prepared using the following process:
In step 1, in a first appropriately sized tank (or vessel), a premix is formed by adding 7.0% first polyol solvent (i.e., propylene glycol), active oils, organic acid buffer benzoic acid, and flavor with mixing until homogeneous and uniform. In step 2, in a second appropriately sized tank (or vessel), a second premix is formed by adding to a preservative sodium benzoate, surfactants (Poloxamer 407, sodium lauryl sulfate, lauramidopropyl betaine, cocoamidopropyl betaine), and sweeteners to water with mixing until homogeneous and uniform. In step 3, the kiwi extract (*Pichia anomala* extract) is added to the premix in step 2, along with dye. In step 4, the first premix is added to the second premix with mixing until homogeneous and uniform. In step 5, the sugar alcohol is added as the final component to the mixture of the two premixes with mixing until the final mixture is homogeneous and uniform.

**Table 2**

| | **Formula 12** | **Formula 13** | **Formula 14** | **Formula 15** |
|---|---|---|---|---|
| **Ingredients** | **(% w/w)** | **(% w/w)** | **(% w/w)** | **(% w/w)** |
| Propylene glycol | - | - | - | - |
| Ethanol | 15.0 | 18.2 | 10.0 | 25.0 |
| L-Menthol | 0.0413 | 0.0413 | 0.0413 | 0.0413 |
| Thymol | 0.0620 | 0.0620 | 0.0620 | 0.0620 |
| Methyl salicylate | 0.0641 | 0.0641 | 0.0641 | 0.0641 |
| Eucalyptol | 0.0895 | 0.0895 | 0.0895 | 0.0895 |
| Flavor | 0.2000 | 0.1000 | 0.2000 | 0.2200 |
| *Pichia anomala* extract A | 2.0000 | - | - | - |
| *Pichia anomala* extract B | - | 0.5000 | - | - |
| *Pichia anomala* extract C | - | - | 1.0000 | - |
| *Pichia anomala* extract D | - | - | - | - |
| *Pichia anomala* extract E | - | - | - | 0.1000 |
| Sorbitol (70% solution) | 10.0 | 19.6 | 19.6 | 15.0 |
| Sodium lauryl sulfate | - | - | - | - |
| Poloxamer 407 | 0.2500 | 0.2500 | 0.2500 | 0.2500 |
| Lauramidopropyl betaine | - | - | - | - |
| Cocoamidopropyl betaine | - | - | - | - |
| Sodium saccharine | 0.1100 | 0.1100 | 0.1100 | 0.1100 |
| Sucralose | - | - | - | - |
| Benzoic acid | 0.1200 | 0.1200 | 0.1200 | 0.1200 |
| Sodium benzoate | 0.0350 | 0.0350 | 0.0350 | 0.0350 |
| FD&C Green #3 | 0.00002 | 0.00002 | 0.00002 | 0.00002 |
| Purified Water | QS | QS | QS | QS |

### Methods for making formulations of Table 2

Formulations 12-15 can be prepared using the following process:
Formulations 12-15 (using the inventive process): In step 1, in a first appropriately sized tank (or vessel), a premix is formed by adding equal parts water and alcohol (ethanol) and mixing in active oils, organic acid buffer benzoic acid, preservative sodium benzoate, and flavor with mixing until homogeneous and uniform. In step 2, surfactants (Poloxamer 407), and sweeteners and remaining water is mixed into the premix of step 1 until homogeneous and uniform. In step 3, the kiwi extract (*Pichia anomala* extract) and dye are added to the premix from step 1. In step 4, the sugar alcohol is added as the final component with mixing until the final mixture is homogeneous and uniform.

### Embodiments of the Present Invention

1. A composition for treating the oral cavity comprising:
   i) one or more extracts, or sources of extracts, of the genus *Pichia;* and
   ii) a pharmaceutically or cosmetically acceptable carrier.
2. The composition of embodiment 1 (or, any of the following embodiments), wherein the genus of *Pichia* is selected from *Pichia anomala, Pichia guilliermondii, Pichia norvegensis, Pichia ohmeri* and mixtures thereof.
3. The composition of embodiments 1 and/or 2 (or, any of the following embodiments), wherein the genus of *Pichia* is *Pichia anomala.*
4. The composition of any one of or combination of embodiments 1 to 3 (or, any of the following embodiments), wherein the one or more extracts, or sources of extracts, of the genus *Pichia* are present at a concentration of from about 0.01% to about 100% by weight of the total composition.
5. The composition of any one of or combination of embodiments 1 to 4 (or, any of the following embodiments), wherein the one or more extracts, or sources of extracts, of the genus *Pichia* are present at a concentration of from about 0.05% to about 95% by weight of the total composition.
6. The composition of any one of or combination of embodiments 1 to 5 (or, any of the following embodiments), wherein the one or more extracts, or sources of extracts, of the genus *Pichia* are present at a concentration of from about 0.1% to about 90% by weight of the total composition.
7. The composition of any one of or combination of embodiments 1 to 6 (or, any of the following embodiments), comprising a permeation enhancer.
8. The composition of any one of or combination of embodiments 1 to 7 (or, any of the following embodiments), wherein the permeation enhancer is present at a concentration of from about 0.01% to about 20% (w/v) of the total composition.
9. The composition of any one of or combination of embodiments 1 to 8 (or, any of the following embodiments), wherein the permeation enhancer is present at a concentration of from about 0.1% to 10% (w/v) of the total composition.
10. The composition of any one of or combination of embodiments 1 to 9 (or, any of the following embodiments), wherein the permeation enhancer is present at a concentration of from about 0.25% to 5% (w/v) of the total composition,
11. The composition of any one of or combination of embodiments 1 to 10 (or, any of the following embodiments), wherein the permeation enhancer is selected from polyoxyethylene, polyoxyethylene ethers of fatty acids, sorbitan monooleate, sorbitan monolaurate, polyoxyethylene monolaurate, polyoxyethylene sorbitan monolaurate, fusidic acid and derivatives thereof, EDTA, disodium EDTA, cholic acid, deoxycholic acid, glycocholic acid, glycodeoxycholic acid, taurocholic acid, taurodeoxycholic acid, sodium cholate, sodium glycocholate, glycocholate, sodium deoxycholate, sodium taurocholate, sodium glycodeoxycholate, sodium taurodeoxycholate, chenodeoxycholic acid, urosdeoxycholic acid, saponins, glycyrrhizic acid, ammonium glycyrrhizide, decamethonium, decamethonium bromide, and dodecyltrimethylammonium bromide or mixtures of any of the above.
12. The composition of any one of or combination of embodiments 1 to 11 (or, any of the following embodiments), wherein the *Pichia* genus extract comprises oligosaccharides and polysaccharides having a weight average molecular weight of from about 180 to about 800,000 Da.
13. The composition of any one of or combination of embodiments 1 to 12 (or, any of the following embodiments), wherein the *Pichia* genus extract comprises oligosaccharides and polysaccharides having an average degree of polymerization of from DP 1 to DP 4444.
14. A method for producing/releasing/delivering/excreting mucin from and/or in the oral cavity comprising the step of administering a composition comprising:
   i) one or more extracts, or sources of extracts, of the genus *Pichia;* and
   ii) optionally, a pharmaceutically or cosmetically acceptable carrier.
15. The method according to claim 14, wherein the genus of *Pichia* is selected from *Pichia anomala, Pichia guilliermondii, Pichia norvegensis, Pichia ohmeri* and mixtures thereof.
16. The method according to claim 15, wherein the genus of *Pichia* is *Pichia anomala.*

## Claims

1. A composition comprising:
i) one or more extracts, or sources of extracts, of the genus *Pichia;* and
ii) a pharmaceutically or cosmetically acceptable carrier.

2. The composition according to claim 1, wherein the genus of *Pichia* is selected from *Pichia anomala, Pichia guilliermondii, Pichia norvegensis, Pichia ohmeri* and mixtures thereof, or wherein the genus of *Pichia* is *Pichia anomala.*

3. The composition of claim 1 and/or claim 2, wherein the one or more extracts, or sources of extracts, of the genus *Pichia* are present at a concentration of from about 0.001% to about 100% by weight of the total composition.

4. The composition of any of claims 1 to 3, wherein the one or more extracts, or sources of extracts, of the genus *Pichia* are present at a concentration of from about 0.005% to about 95% by weight of the total composition.

5. The composition of any of claims 1 to 4, wherein the one or more extracts, or sources of extracts, of the genus *Pichia* are present at a concentration of from about 0.01% to about 90% by weight of the total composition.

6. The composition according to any of claims 1 to 5, comprising a permeation enhancer.

7. The composition of claim 6 wherein the permeation enhancer is present at a concentration of from about 0.01% to about 20% (w/v) of the total composition, or wherein the permeation enhancer is present at a concentration of from about 0.1% to 10% (w/v) of the total composition, or wherein the permeation enhancer is present at a concentration of from about 0.25% to 5% (w/v) of the total composition.

8. The composition of claim 6 and/or claim 7 wherein the permeation enhancer is selected from polyoxyethylene, polyoxyethylene ethers of fatty acids, sorbitan monooleate, sorbitan monolaurate, polyoxyethylene monolaurate, polyoxyethylene sorbitan monolaurate, fusidic acid and derivatives thereof, EDTA, disodium EDTA, cholic acid, deoxycholic acid, glycocholic acid, glycodeoxycholic acid, taurocholic acid, taurodeoxycholic acid, sodium cholate, sodium glycocholate, glycocholate, sodium deoxycholate, sodium taurocholate, sodium glycodeoxycholate, sodium taurodeoxycholate, chenodeoxycholic acid, urosdeoxycholic acid, saponins, glycyrrhizic acid, ammonium glycyrrhizide, decamethonium, decamethonium bromide, and dodecyltrimethylammonium bromide or mixtures of any of the above.

9. The composition of any of claims 1 to 8 wherein the *Pichia* genus extract comprises oligosaccharides and polysaccharides having a weight average molecular weight of from about 180 to about 800,000 Da.

10. The composition of any of claims 1 to 9 wherein the *Pichia* genus extract comprises oligosaccharides and polysaccharides having an average degree of polymerization of from DP 1 to DP 4444.

11. The composition of any of claims 1 to 10 wherein the composition is:
(a) provided in the form of a solution, mouthwash, mouth rinse, mouth spray, toothpaste, tooth gel, sub-gingival gel, mousse, foam, denture care product, dentifrice, lozenge, chewable tablet, dissolvable tablet, or dry powder; or
(b) incorporated into or onto floss, dissolvable strips or films, or integrated into or onto a device or applicator for oral use.

12. A composition for use in treating oral disease, wherein the composition is as claimed in any of claims 1 to 11.

13. The composition for use of claim 12 wherein the composition is contacted with a surface of the oral cavity which produces/releases/delivers/excretes mucin from and/or in the oral cavity, disrupts a biofilm on a surface of the oral cavity, removes biofilm from a surface, reduces bacterial attachment to a surface, and/or inhibits plaque.

14. The composition for use of claim 12, wherein the oral disease is selected from decalcification of teeth, periodontitis and dental caries.

15. A non-therapeutic method of treating the oral cavity using a composition as claimed in any of claims 1 to 11.
